# EUROPEAN PATENT SPECIFICATION

(11) **EP 1 161 948 B1**
(45) Date of publication and mention of the grant of the patent: **22.12.2004**
(21) Application number: 01303728.8
(22) Date of filing: 24.04.2001
(51) Int. Cl.: A61K 31/4045, A61K 9/00, A61K 9/16, A61K 9/127, A61P 25/00, A61P 7/10, A61P 9/10, A23L 1/29

(54) **Pharmaceutical or food composition for treatment of brain edema**
Pharmazeutische Zusammensetzung und Nahrungsmittel zur Behandlung von Gehirnödem
Composition pharmaceutique et nutritive pour traiter l'oedème cérébral

(30) Priority: 24.04.2000 US 556701
(43) Date of publication of application: 12.12.2001
(73) Proprietor: Ajinomoto Co., Inc., Tokyo 104-8315 (JP)
(72) Inventor: Nishino, Hitoo, Nagoya-shi, Aichi (JP); Torii, Kunio, c/o Central Research Laboratories, Kawasaki-ku, Kawasaki-shi, Kanagawa-ken (JP); Uneyama, Hisayuki, c/o Central Research Lab., Kawasaki-ku, Kawasaki-shi, Kanagawa-ken (JP)
(74) Representative: Nicholls, Kathryn Margaret

(56) References cited:
- WO-A-97/20555
- WO-A-98/21947
- US-A- 4 945 103
- US-A- 5 500 225
- US-A- 6 075 045
- DATABASE WPI Section Ch, Week 199941 Derwent Publications Ltd., London, GB; Class B02, AN 1999-491878 XP002213650 & KR 98 049 239 A (YOOHAN Q FOOD JH), 15 September 1998 (1998-09-15)
- DATABASE WPI Section Ch, Week 200150 Derwent Publications Ltd., London, GB; Class B02, AN 2001-457909 XP002213649 & CN 1 164 422 A (LUDE LLC BEIJING), 12 November 1997 (1997-11-12)
- QI WENBO ET AL: "Melatonin reduces lipid peroxidation and tissue edema in cerulein-induced acute pancreatitis in rats." DIGESTIVE DISEASES AND SCIENCES, vol. 44, no. 11, November 1999 (1999-11), pages 2257-2262, XP008008300 ISSN: 0163-2116
- COSTANTINO GIUSEPPINA ET AL: "Protective effects of melatonin in zymosan-activated plasma-induced paw inflammation." EUROPEAN JOURNAL OF PHARMACOLOGY, vol. 363, no. 1, 11 December 1998 (1998-12-11), pages 57-63, XP001113117 ISSN: 0014-2999
- GORGULU ASKIN ET AL: "Effect of melatonin on cerebral edema in rats." NEUROSURGERY (BALTIMORE), vol. 49, no. 6, December 2001 (2001-12), pages 1434-1442, XP008008308 December, 2001 ISSN: 0148-396X
- SARRAFZADEH A S ET AL: "Neuroprotective effect of melatonin on cortical impact injury in the rat." ACTA NEUROCHIRURGICA. AUSTRIA 2000, vol. 142, no. 11, 2000, pages 1293-1299, XP001113116 ISSN: 0001-6268
- MESENGE: "protective effects of melatonin..." J. PINEAL RES., vol. 25, 1998, pages 41-46, XP008008633

## Description

The present invention relates to a composition for the treatment or prevention of brain edema. More particularly, it relates to a pharmaceutical or food composition for the treatment or prevention of brain edema comprising melatonin as an active ingredient. Additionally, it relates to the use of melatonin in the preparation of the above composition. Finally, it relates also to the use for the manufacture of a medicament for the treatment or prevention of brain edema with the above composition.

Brain edema refers to a condition where fluid is excessively accumulated in intercellular spaces or in cells of brain tissue resulting in swelling of brain tissue. The swelling of brain tissue in the limited cranial space increases intracranial pressure. Thus, brain edema is generally associated with increased intracranial pressure.

Brain edema can be etiologically classified into "vasogenic brain edema" and "cytotoxic brain edema" (I. Klatzo, J. Neuropatho. Exp. Neurol., 25: 1-14, 1967).

Vasogenic edema is caused by injury of cerebral blood vessels. Injury of cerebral vessels and capillaries modifies and deteriorates vascular permeability. When vascular permeability is modified and deteriorated, fluid migrates into intracellular spaces of brain. The migration of fluid increases the fluid content in the intracellular spaces. The vasogenic edema is typically found in brain tumor and cerebral hemorrhage.

Cytotoxic edema is caused by injury of cells. Injury of cells modifies and deteriorates the permeability of cell membrane. When the cell membrane permeability is modified and deteriorated, fluid is allowed to migrate into cells. The migration of fluid increases the fluid content in the cells. The cytotoxic edema is typically found in hypoxia, toxipathy (induced by arsenic, carbon oxide and the like) and metabolic disorders (diabetic coma, uremia and the like).

Apart from the above two types of edema, brain edema caused by brain ischemia or deficient cerebral blood flow is called "ischemic brain edema". While, brain edema caused by cerebral recirculation after brain ischemia or cerebral blood flow deficiency is called "post-ischemic brain edema". The modification of vascular or cellular permeability would also be involved in the onset of the edema of this classification.

The increased intracranial pressure caused by brain edema gives rise to a secondary cerebral disorder such as disturbance of cerebral blood flow, ischemia, hypoxia, and cerebral hernia. The secondary cerebral disorder triggers additional deterioration of vascular or cellular permeability, and the additional deterioration of vascular or cellular permeability causes additional brain edema, extending edema by edema itself. Such an extension of edema by edema itself looks like a forest fire where a primary fire is the cause of an additional fire (see I. Klatzo & F. Seitelberger (eds): Brain Edema, Springer-Verlag, New York, 1967).

Thus, the "forest fire-like" extension is inherent and characteristic in brain edema that generates in a rigid and limited cranial space which essentially differs from the edemas of the other organs. Due to the "forest fire-like" extension, brain edema is a severe mortal disease.

The treatment of brain edema relies on the administration of a hypertonic solution, a steroid, a diuretic and an adjuvant such as a thrombolytic and a microcirculation improver. The hypertonic solution mainly comprises glycerol or mannitol (for example, 10% of glycerol, 5 % of fructose and 0.9 % of NaCl) which promotes the movement of a fluid in a brain tissue into a blood vessel by increasing serum osmotic pressure. The steroid is considered to exhibit an anti-brain edema effect by reinforcing a cell membrane.

With increase in the aged population, the patients suffering from brain diseases (such as cerebral thrombosis, cerebral embolism and cerebral infarction) which injure brain tissues and then vascular or cellular permeability and cause brain edema will be increased. Consequently, the treatment and prevention of brain edema is a problem to be urgently solved.

Melatonin (N-[2-(5-methoxy-lH-indol-3-yl)ethyl]acetamide) is secreted from the pineal gland which is one of the neurohormonal organs and influences the formation of a diurnal rhythm (for example, Chem. & Eng. News, 45: 40, 1967).

Melatonin is used for the treatment of disorders of a diurnal rhythm such as a sleep disorder and emotional disorder caused by for example jet-lag or night work (for example, Barchas et al., Nature 214: 919, 1967 and A. Miles et al., CRC Crit. Rev. Clin. Lab. Sci., 25: 231-253, 1987).

Melatonin has an anti-oxidative activity. For example, it prevents in vitro oxidative deterioration by oxygen free radicals in various biocomponents (R. J. Reiter et al., Life Sci., 60(25), 2255-2271, 1997).

R. J. Reiter describes that one of the potential major causes of age-related brain deterioration is toxic free radicals formed by aerobic metabolism in brain. According to R. J. Reiter, such brain deterioration could be reduced by the anti-oxidative activity of melatonin (R. J. Reiter, FASEB J., 9: 526-533, 1995).

Further, it is pointed out that administered melatonin inhibits the production of NO in the brain after transient ischemia/recirculation and reduces brain injury caused by the free radicals (J. M. Guerrero et al., J. Pineal Res., 23: 24-31, 1997).

Sunghee Cho et al., Brain Res., 755(2), 335-338, 1978 reports that intraperitoneally administered melatonin, especially prior to cerebral ischemia or during recanalization, protects CA1 hippocampal neurons from ischemic injury.

It has been demonstrated with the rat model of brain having ischemia induced by ligating a middle cerebral artery that brain necroses in rats having no detectable level of melatonin after pinealectomy are significantly larger than in normal rats (Hari Manev et al., FASEB J, 10(13), 1546-1551, 1996).

WO 97/20555 discloses that a mild motor dysfunction resulting from brain ischemia of the foot-fault rate of 0.01 can be lowered to the foot-fault rate of 0.002 by administration of a "rescue solution" containing melatonin, kynurenine and other agents to the brain ischemic rat. The foot-fault rate of the group without the administration of the rescue solution was 0.005. The foot-fault rate is determined according to Hernandez-Schallert foot-fault test wherein rats are forced to walk on a bar 3 to 6 cm in diameter and the number of rats which slip is counted. The foot-fault rate is a proportion of rats which slip after ischemia to rats which slip before ischemia.

On the other hand, it has been known that orally administered melatonin migrates in blood (A. L. Elizabeth et al., J. Clin. Endocrinol. Metab., 61: 1214-1216, 1985; O. Vakkuri et al., Life Sci., 37: 489-495, 1985; M. Aldhous et al., Br. J. Clin. Pharmacol., 19: 517-521, 1985; and F. Waldhauser et al., Neuroendocrinology, 39: 307-313, 1984).

It has been known that intravenously administered melatonin migrates in brain (P. A. Vitte et al., Pineal Res. 5: 437-453, 1988 and D. L. Baris et al., Int. J. Rds. Appl. Instrum. [B] 18: 357-362, 1991) (J. Pineal res. vol. 25, 1998, pages 41 - 46).

As to edema in other tissues than brain, S. Bertuglia et al., Cardiovascular Research, 31: 947-952, 1996 describe that the administration of melatonin reduces edema caused by ischemia-reperfusion of a microcirculation in a cheek pouch of a hamster. According to this article, melatonin reduces the increase of permeability of capillaries caused by free radicals (produced by topically exposing to hypoxanthine-xanthine oxidase). S. Cruzzocrea et al., J. Pineal Res., 23: 106-116, 1997 demonstrate that the administration of melatonin suppresses carrageenan-induced inflammatory paw swelling in rats Also US 4 945 103 ; "Digestive Diseases and Sciences" (11 - 1999), 44 (11), 2257 - 2262 ; and "European Journal of Pharmacology" 11/12/98, 363 (1) 57-63, disclose that melatonin is useful against paw edema. It is suggested that control of formation of an inducible NO synthase and a scavenging action of a free radical, peroxynitrite is involved in the above-discussed inhibition. Y. Oyanagui, Inflammation, 21: 643-654, 1997 demonstrates that various antioxidants including melatonin enhance or prolong suppression by dexamethasone of ischemic or histamine-induced paw edema. Further, W. Qi, et al, Dig. Dis. Sci., 44: 2257-2262, 1999 describe that melatonin reduces an acute pancreatitis (pancreatic edema) induced by cerulein in rats. It is supposed that the radical scavenging action of melatonin is involved in the above reduction of edema.

The above local inflammatory edema would be caused also by the modification in vasopermeability (caused by inflammation) or the modification in cell membrane permeability (caused by free radicals). However, cerebral vasopermeability is controlled by blood-brain barrier which is absent in blood vessels in other organs. Particularly, astroglia is involved in the blood-brain barrier, which is also absent in blood vessels in other organs. Studies of brain edema in view of the brain specific vasopermeability have not been satisfactorily conducted.

Further, a study from the viewpoint of a "forest fire-like" extension of brain edema, which is caused by the presence of brain tissue in the limited space via the brain-specific blood-brain barrier and astroglia, has not been fully conducted.

In order to treat and prevent brain edema more effectively, a further study from a new and different viewpoint from above will be necessary.

Accordingly, an object of the invention is to provide a useful and effective means for the treatment or prevention of brain edema.

One aspect of the invention relates to a pharmaceutical or food composition for the treatment or prevention of brain edema comprising an effective amount of melatonin for the treatment or prevention.

Another aspect of the invention is the use of melatonin for the preparation of a pharmaceutical or food composition for the treatment or prevention of brain edema comprising an effective amount of melatonin for the treatment or prevention.

Embodiments of the invention are described below, by way of example only, and with reference to the accompanying drawings of which:

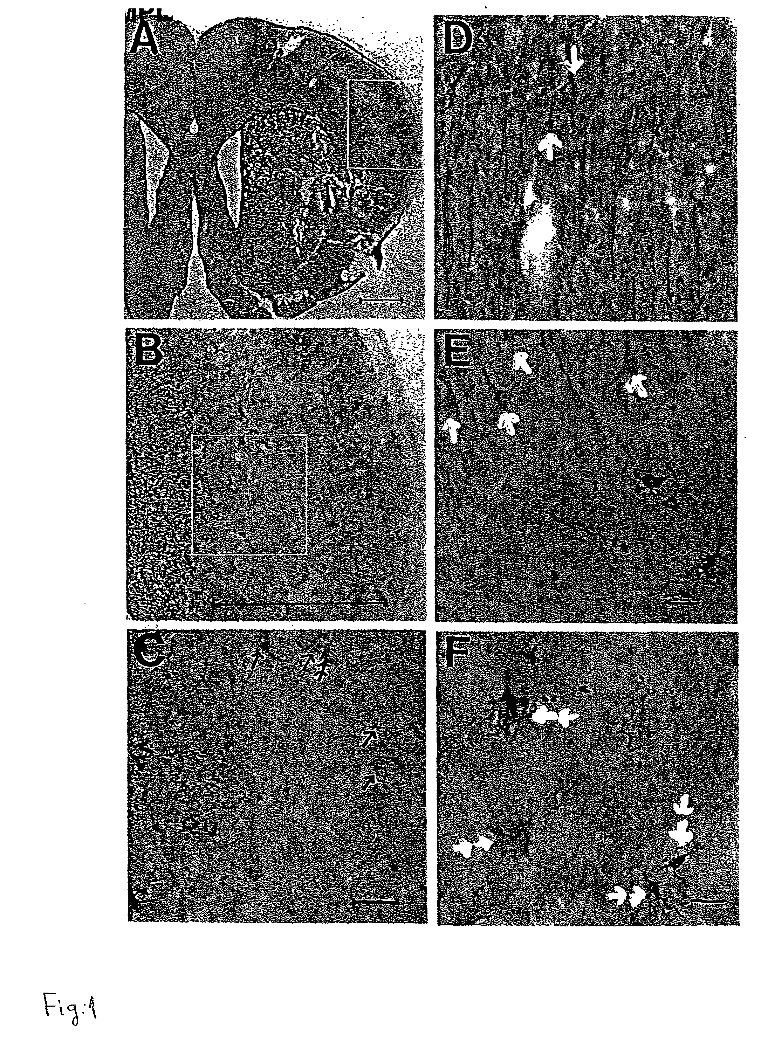

Fig. 1 shows photographs of specimens of a brain after ischemia. Photograph A is a micrograph of a specimen stained with GFAP. Photograph B is an amplification of an area enclosed within the square marked in photograph A. Photograph C is an amplification of an area enclosed within the square marked in photograph B. Photograph D is an amplification of an area enclosed within a square in photograph A and shows an area of cortex near the penumbra at a higher magnification. Figures 1E and 1F show cerebral cortex in adjacent sections of Fig. 1A. The scale as indicated by the scale bar at the lower right in each photograph is 1 mm in Fig. 1A and 1B, 100µm in Fig. 1C, and in each of photographs D to F is 50 µm. A white single arrow, →, as marked in Figs. D and E shows a neuron and a white double arrow, →→, marked in Fig. F designates both a neuron and an astroglia.

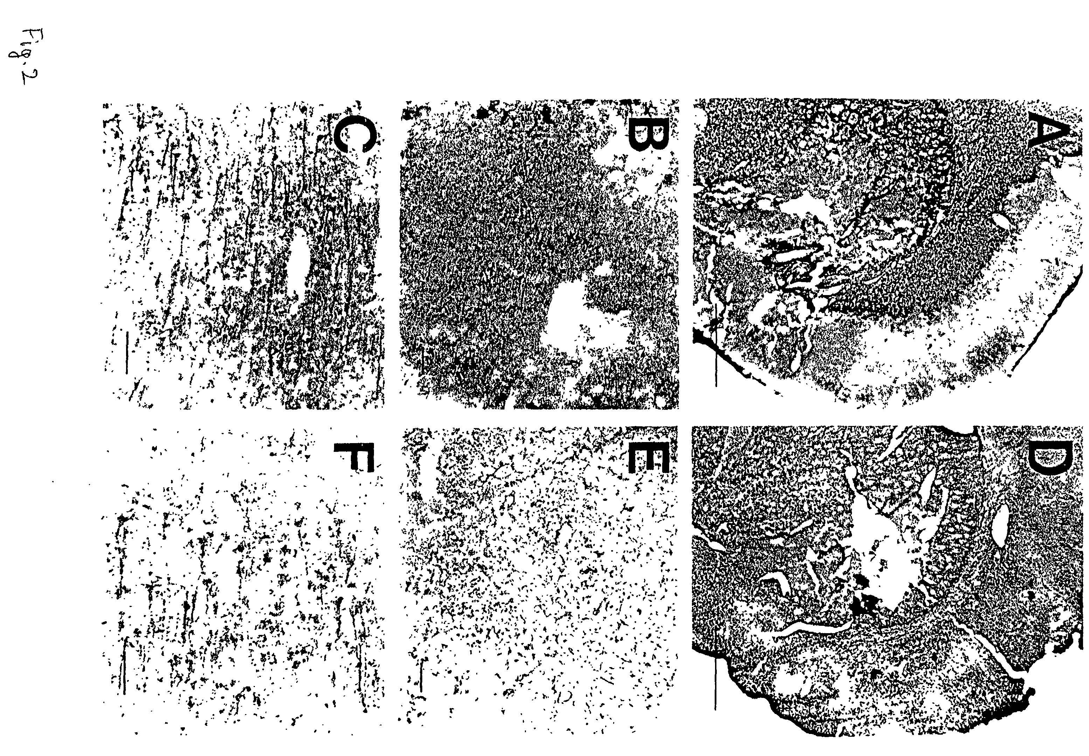

Fig. 2 shows photographs of specimens of ischemic brains of a melatonin-treated rat and a melatonin-non-treated (control) rat. Photographs A, B and C are micrographs of specimens of a melatonin-non-treated rat administered with saline. Photographs D, E and F are micrographs of specimens of a rat administered with melatonin. The specimens of Photographs A, B, D and E were stained with DFAP. The specimens of Photographs C and F were stained with MAP-2.

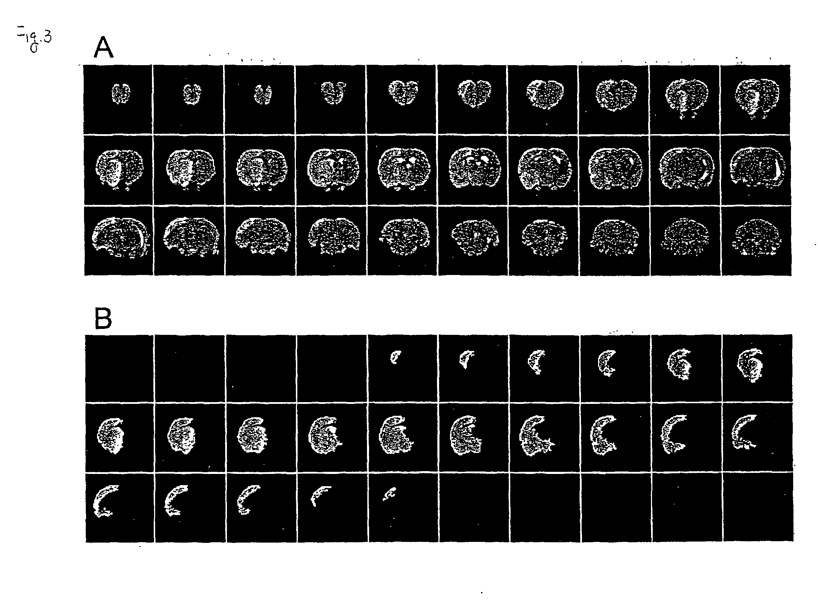

Fig. 3 shows 30 consecutive T₂-weighted magnetic resonance images of a brain of an ischemic rat administered with saline (control). Photographs A show each whole area of slices. Photographs B show each edematous area from the whole areas of Photographs A. Images were aligned from rostral to caudal slices. Slice thickness was 0.6 mm, and total covered thickness was 18.0 mm.

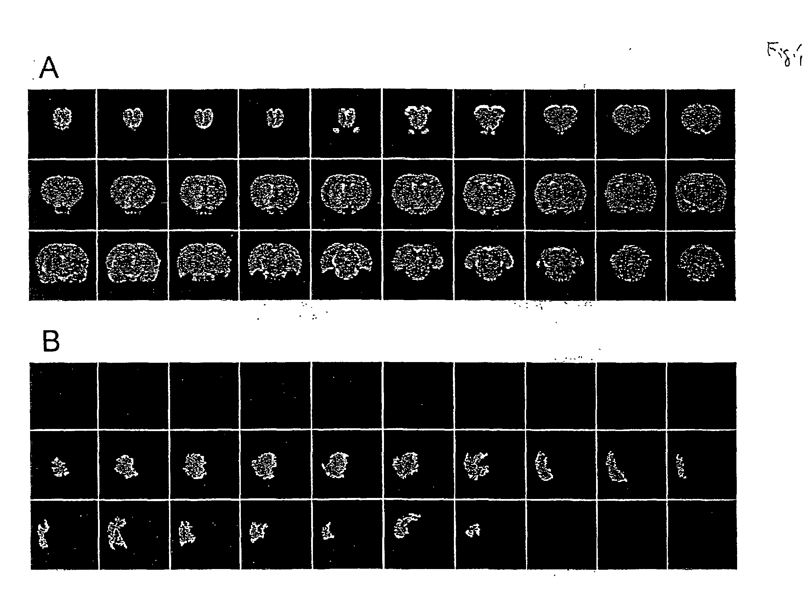

Fig. 4 shows 30 consecutive T₂-weighted magnetic resonance images of a brain of an ischemic rat administered with melatonin. Photographs A show each whole area of slices. Photographs B show each edematous area from the whole areas of Photographs A. Images were aligned from rostral to caudal slices. Slice thickness was 0.6 mm, and total covered thickness was 18.0 mm.

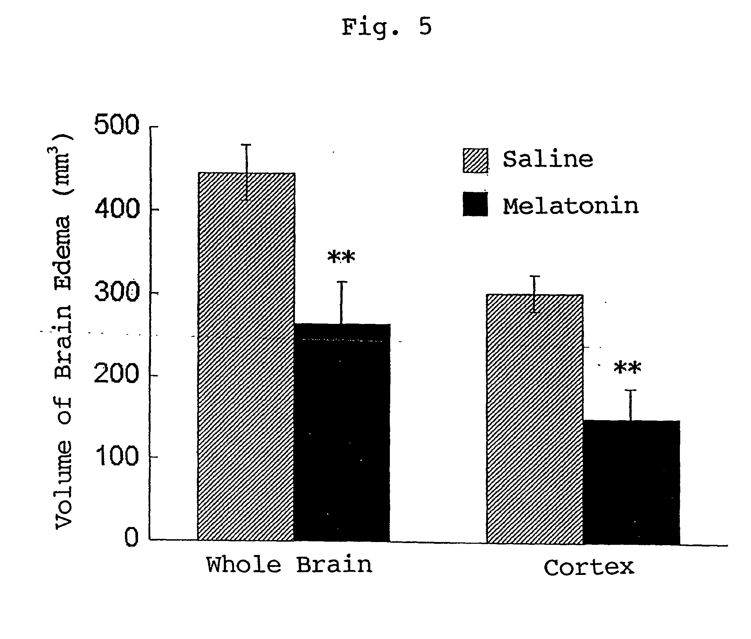

Fig. 5 shows the volume of brain edema of ischemic rats administered with melatonin determined with the magnetic resonance images of Figs. 3 and 4 comparing with the volume of brain edema of ischemic rats administered with saline.

During our study concerning the treatment and prevention of brain edema, our attention was directed to a blood-brain barrier deeply involved in a "forest fire-like" extension of brain edema and inherent in a cerebral blood vessel, especially astroglia characteristic in a blood-brain barrier. Then, our study was directed to the blood-brain barrier, especially injury of astroglia, its protection and a treatment of injured astroglia.

Two findings have been made as a result of the above study, which are the bases of the invention.

The first finding is that brain ischemia preferentially injures astroglia as compared with neurons (Fig. 1). The injury of astroglia will be directly associated with an injury of a blood-brain barrier, that is, a modification in a permeability of a cerebral blood vessel. Then, the first injury of astroglia will cause brain edema, and the brain edema will additionally generate deficiency of cerebral blood flow. The additional deficiency of cerebral blood flow will secondarily injure other healthy astrocytes expanding the brain edema.

The second finding is that melatonin preferentially protects astroglia from an injury caused by a poison or ischemia and preferentially cures an injured astroglia (Fig. 2). The protection and curing of astroglia by melatonin will directly prevent the vascular permeability from injury and cures the injury.

As the results of further studies based on the above two findings, we have found that melatonin treats and prevents brain edema, and inhibits a "forest fire-like" extension of brain edema through its capacity to protect astroglia from injury and to cure injured astroglia.

The preferential activity of melatonin towards astroglia cannot be explained from the anti-oxidative activity of melatonin suggested to be involved in a local inflammatory edema as described in the references mentioned above.

The first group of subjects to be administered with the pharmaceutical or food composition of the invention comprising melatonin is the group of patients suffering from brain edema. According to our study, in the first group of the subjects, melatonin treats astroglia injured by ischemia, edema or other causes and treats a blood-brain barrier so that edema is cured. Melatonin prevents or reduces secondary injury of the blood-brain barrier caused by secondary injury of astroglia caused by brain edema so that a "forest fire-like" extension of brain edema is suppressed. Accordingly, the composition of the invention is used for the treatment of brain edema in the first group of the subjects.

The second group of subjects to be administered with the pharmaceutical or food composition of the invention is the group of subjects who are judged by clinicians to have the risk of suffering from brain ischemia. According to our finding, such subjects have the risk of injuring their astroglia and blood-brain barrier. In the second group of the subjects, melatonin will protect the astroglia and blood-brain barrier from injury caused by brain ischemia so that onset of brain edema is prevented. Accordingly, the composition of the invention is used for the prevention of brain edema in the second group of the subjects.

The subjects having the risk of suffering from brain ischemia include, but are not limited to, subjects suffering from cerebral thrombosis, cerebral embolism, cerebral infarction, cerebral hemorrhage, subarachnoid hemorrhage, transient brain ischemia, hyperlipemia, hypertension, cardiac arrest or brain contusion.

Diagnosis and monitoring of brain edema are conducted by a clinician based on information including a progress of conditions such as an increase in intracranical pressure, a CT scanning, a magnetic resonance imaging (MRI) and the like. That is, the brain edema of this invention depends on and is defined as the result of a clinician's judgement based on the above diagnosis or monitoring.

Melatonin, as used herein, includes melatonin encapsulated in an encapsulating matrix, a liposome.

Melatonin encapsulated in an encapsulating matrix is gradually released from the matrix into blood so that its residence time in blood is apparently increased.

The encapsulating matrix comprises cyclodextrin and pharmaceutically acceptable biodegradable synthetic polymers such as polylactic acid, a copolymer of lactic acid and glycol, poly-β-hydroxybutyric acid and the like. Cyclodextrins attached onto a pharmaceutically acceptable synthetic polymer are included in the definition of cyclodextrin.

When melatonin is encapsulated in a liposome, its residence time in blood can be also increased.

As methods for the preparation of liposomes, various methods such as a vortex method (Bangham AD et al., Methods Membr. Biol., 1: 1-20, 1974), an ultrasonic treatment method (Johnson SM et al., Biochim. Biophys. Acta, 233: 820-826, 1971), an ethanol injection method (Kremer JMH et al., Biochemistry, 16: 3932-3941, 1980), a french press method (Hamilton et al., J. Lipid Res. 21: 981-982, 1980), a cholic acid removal method (Enoch HG et al., Proc. Natl. Acad. Sci., 76: 145-149, 1979), an ether injection method (Deamer DN, Ann, N.Y. Acad. Sci., 308: 250-258, 1987), a freeze-thaw method (Papahadjopoulos D et al., Biophim. Biophys. Acta, 394: 483-491, 1975) and a reverse phase evaporation method (Szoka F et al., Proc. Natl. Acad. Sci. USA, 75: 4191-4198, 1978) and the like have been known.

The composition of the invention may be either a pharmaceutical composition or a food composition to be administered or served to the subject. The pharmaceutical and food compositions essentially contain melatonin in an amount effective for treatment or prevention of brain edema.

It is desirable to administer or serve the composition of the invention to the subject as soon as possible when brain ischemia was found. If the administration or serving is delayed, brain edema will be generated and extended. By the early administration or serving, it is possible to prevent an onset of brain edema.

For prevention of brain edema, the composition of the invention can be administered or served every day or at a predetermined interval depending on a condition or state of a disease giving the risk of brain edema such as cerebral thrombosis or cerebral embolism. The administration or serving of the composition of the invention is continued until the risk of an onset of brain edema is lowered to a desirable level, and depends on a clinician's judgement.

In treatment of brain edema, the administration or serving of the composition of the invention is continued until the brain edema is cured or is lowered to a desired level. It is stopped also by a clinician's judgement.

The amount of melatonin to be administered or served to the subjects depends on various factors such as the sex of the subject, the age of the subject, the body weight of the subject, the diet of the subject, the administration route applied, the condition of the brain edema, the degree of the risk of inducing brain ischemia, the condition of the diseases which may give the subject brain ischemia (such as cerebral thrombosis and cerebral embolism), and the condition of circulatory systems of the subject. The amount is determined by a clinician.

When the composition of the invention is administered or served for the prevention of brain edema, the daily dose or amount of melatonin is determined such that a daily blood concentration of melatonin ranges from 1 ng/ml to 100 µg/ml. When the composition of the invention is administered or served for the treatment of brain edema, its daily dose is determined such that a daily blood concentration of melatonin ranges from 10 ng/ml to 300 µg/ml.

Availability of melatonin varies whether melatonin is administered orally or by intravenous injection, or whether melatonin is encapsulated or not. The availability varies also with the dosage form of oral administration. Therefore, the daily dose of the composition of the invention to be administered varies depending on the administration conditions as above. For example, in case of oral administration of melatonin, an oral dose of about 0.8 mg/kg-body weight would be necessary to give 100 ng/ml of blood concentration of melatonin.

The expression "daily blood concentration" as used above means the total blood concentration of melatonin administered per day. For example, when the blood concentration by the first administration reaches 50 µg/ml and the blood concentration by the second administration on the same day reaches 30 µg/ml, the daily blood concentration is calculated to be 80 µg/ml. It is unnecessary to determine the daily blood concentration by practically measuring the blood concentration of melatonin. A clinician or a specialist will be able to estimate the daily blood concentration of a specific clinical case.

The above-mentioned blood concentration of melatonin is referred to melatonin in a free form. In case melatonin is encapsulated in an encapsulating matrix or a liposome, its apparent residence time in blood is longer since melatonin in an encapsulated form is gradually released in blood. Thus, the blood concentration of melatonin in the encapsulated form may be lower than that in melatonin in the free form.

The pharmaceutical composition of the invention is administered by various routes, such as permucosally (sublingually, intranasally, oral mucosally and the like), orally, enterally, percutaneously, intravenously, by aspiration, by suppository or by instillation. The administration route is determined depending on the amount of melatonin to be administered, conditions of a patient or a subject and the like. It is determined by a clinician.

In addition to melatonin, the pharmaceutical composition of the invention contains one or more of pharmaceutical carriers. The pharmaceutical carrier should be pharmaceutically acceptable. The pharmaceutical carrier to be used mainly depends on the dosage form.

When the pharmaceutical composition of the invention is orally administered, the pharmaceutical carrier may be a binder (such as tragacanth gum, acacia, corn starch and gelatin), a vehicle (such as potassium diphosphate), a disintegrator (such as corn potato, potato starch and alginic acid), a lubricant (such as magnesium stearate), a sweetening agent (such as sucrose), a dye, a flavoring agent (such as orange flavor), and a solvent (such as water, ethanol and glycerol). The pharmaceutical composition for oral administration may contain a pharmaceutically acceptable antioxidant (such as cysteine, glutathione, ascorbic acid, sodium metasulfite, and sodium bisulfite) as a pharmaceutical carrier.

The pharmaceutical composition for injection of the present invention may be a sterile powder composition, a freeze-dried powder composition or the like which can be used by merely dissolving in a sterile water.

A diluent or a solvent such as a sterile water, an isotonic saline and a pH buffer can be used as a pharmaceutically acceptable carrier used in the pharmaceutical composition for injection. An aqueous ethanol may be used as the solvent.

The pharmaceutical composition for injection of the invention may contain, as a pharmaceutical carrier, saccharides (such as glucose, mannitol, and dextran), polyhydric alcohols (such as glycerol), and/or inorganic salts (such as sodium salts and magnesium salts). Further, it may contain a pharmaceutically acceptable antioxidant such as cysteine, glutathione, ascorbic acid, sodium metasulfite, and sodium bisulfite.

Pharmaceutical carriers contained in dosage forms for other administration routes such as intranasal, aspiration or percutaneous administrations are known for those skilled in the art.

The dosage forms and the pharmaceutical carriers mentioned above are known and described in, for example, Reimington's Pharmaceutical Science, ed. 16 (1980), Mack Publishing Company.

The pharmaceutical composition of the invention may contain any nutritional agent in addition to melatonin. For example, when the pharmaceutical composition of the invention is administered orally or parenterally, it may contain nutrients such as amino acids, vitamins, lipids, and glucose. When the pharmaceutical composition of the invention is administered by instillation, it may contain nutrients such as glucose, vitamins, amino acids, and lipids.

Further, the pharmaceutical composition of the invention may contain one or more therapeutic agents conventionally used in the treatment of brain edema. The examples of the therapeutic agent for brain edema include hypertonic solutions such as glycerol and mannitol; steroids such as dexamethasone; diuretics such as furosemide and acetazol; and adjuvants such as an thrombolytic agent, microcirculation improvers, and urinastin and gabexate mesilate stabilizing cell membranes.

In addition, the pharmaceutical composition of the invention may contain therapeutic agents for the treatment of diseases such as cerebral thrombosis, cerebral embolism, and a hypertensive encephalopathy. The known therapeutic agents for the treatment of cerebral embolism include an anti-edema, an anticoagulant, a thrombolytic agent, a calcium antagonist and the like. The known therapeutic agents for the treatment of cerebral thrombosis include an anti-edema, an anti-platelet agent, a calcium antagonist and the like.

The pharmaceutical composition of the invention for oral administration is preferably in the form of sustained release. For the sustained release, standard sustained release preparations such as a preparation having a gel coating and a preparation having a multiple coating and preparations for local release such as a preparation capable of rupturing in a pylorus or a preparation capable of foaming in a duodenum are well known. Examples of the composition for oral administration include tablets, pills, capsules, ampoules, folded powders, elixirs, suspensions, syrups and the like.

The pharmaceutical composition of the invention for per rectal administration may be in the form of a suppository. Various forms of suppositories are well known.

When the composition of the invention is a food composition, any food may be used since melatonin is tasteless and stable against heat and enzymes under cooking conditions. The food includes cooked foods such as a hamburger and soup as well as uncooked foods such as a fruit juice. Cold foods such as an ice cream, emulsified foods such as a mayonnaise, gelled foods such as a custard pudding and a jelly and fermented food such as yogurt are included in the food for the food composition of the invention. Foodstuffs, for example, seasonings such as a tomato sauce, a bouillon and a soy sauce are also included in the food for the food composition of the invention.

Melatonin can be added to the food in a pure form or in a mixed form with one or more food additives. The mixed form may be tablet, granule, powder, extrudate, emulsion, gel or the like. The examples of the food additive are disintegrators (such as starch), extenders (such as starch, protein-lyzate, casein, sugar, and glucose), solvents (such as edible fat or oil, ethanol and water), emulsifiers for W/O or O/W emulsion, and gel-forming materials.

Addition of melatonin into a food may be conducted at any suitable time. For example, melatonin may be added in a raw foodstuff before cooking such as a minced meat, a cooked food such as a bouillabaisse and a stew, or milk before preparation of yogurt. Alternatively, melatonin may be added in a food before cold storing or freezing.

The amount of melatonin to be added in a food is such that the above mentioned daily dose of melatonin is satisfied. The food composition of the invention can be served to the subject one, two or three times a day, with or without administration of the pharmaceutical composition of the invention.

Embodiments of the invention will be described with reference to the following examples which are included herein for the purposes of illustration only.

### Example 1

### (1) Brain ischemia preferentially injures astroglia.

Eight to ten-week-old Wister male rats having the body weight of 250 to 300 g were used.

Under halothane anesthesia, a 24G plug was inserted from a right external carotid artery of each rat so as to arrive to an origin of its middle cerebral artery of Willis's circle through its internal carotid artery. Immediately the anesthesia was stopped and the rat was dehypnotized. After one hour, the plug was taken out under halothane anesthesia, thereby a blood flow was recanalized.

After 11 days from an onset of brain ischemia, the brain of each rat was fixed with an aqueous 4% paraformaldehyde solution to prepare a brain specimen. Then, the brain specimen was stained according to the standard ABC method using an anti-GFAP antibody (GFAP staining; astroglia were stained)

As shown in photograph A and photograph B corresponding to an amplification of an area enclosed with a square in photograph A, astrocytes in an ischemic core area were fallen out and gliosis (i.e. an overgrowing of astroglia) was observed in its penumbra area (i.e. a lateral area of corpus striatum and a mantle of cerebral cortex). Photograph C corresponding to an amplification of an area enclosed with a square in photograph B showed that in an ischemic core area of cerebral cortex, astroglia was killed, but neurons were alive. Photograph D corresponding to an amplification of an area enclosed with a square in photograph A showed that in a penumbra area of cerebral cortex, astroglia was killed, but neurons (marked by white single arrows) were alive. Photographs E and F show cerebral cortex, in which → represents a neuron and →→ represents both a neuron and an astroglia. Photograph E showed that astroglia were killed, but neurons were alive. Photograph F showed that both neurons and astroglia was alive.

These results demonstrate that brain ischemia preferentially injures astroglia to neurons, i.e. that neuron is not injured directly by brain ischemia.

These results strongly suggest that brain edema resulting from brain ischemia is caused by a modification in cerebrovascular permeability resulting from the injury of astroglia and that a secondary modification in cerebrovascular permeability resulting from a secondary injury of astroglia caused by a secondary lowering or deficiency of a cerebral blood flow caused by the original edema is involved in a "forest fire-like" extension of brain edema.

### (2) Melatonin protects astroglia from the injury due do brain ischemia (in vivo).

Ischemic rats in Example 1 were divided into two groups. In the first group (non-melatonin administration group), a physiological saline solution was injected to a stomach of each rat using a sound for 10 days from the first day (24 hours after the recanalization of the blood flow). In the second group (melatonin administration group), a melatonin-containing physiological saline solution was similarly administered in an amount of 6 mg of melatonin per kg of rat.

Specimens of brain in the non-melatonin administration group (photographs A, B and C) and in the melatonin administration group (photograph D, E and F) were made in the same manner as described in Example 1. These brain specimens were stained using a GFAP staining (photographs A, B, D and E) or a MAP-2 staining MAP-2 stains neurons.

Photograph B corresponds to an amplification of an area enclosed with a square in photograph A. Photograph E corresponds to an amplification of an area enclosed with a square in photograph D.

The GFAP staining demonstrated that falling out of astroglia in an ischemic core area and gliosis in its penumbra area were present in the non-melatonin administration group and that the falling out of astroglia and the gliosis were reduced by the administration of melatonin in the melatonin administration group.

The MAP-2 staining showed that neurons were alive in both the non-melatonin administration group (photograph C) and the melatonin administration group (photograph F), but the number of the living neurons in the melatonin administration group was higher than that in the non-melatonin administration group.

### (3) Melatonin protects a blood-brain barrier from the injury caused by brain ischemia.

An IgG diapedesis area (area stained with an anti-IgG antibody) from a blood vessel in each of cerebral cortex and corpus striatum was observed using the NIH imaging system in the non-melatonin administration group and the melatonin administration group of Example 2. Results are shown in Table 1.

**Table 1**

| group | population | IgG diapedesis area (mm²; average ± SE) | |
|---|---|---|---|
| | | cerebral cortex | corpus striatum |
| non-elatonin administration | 11 | 14.5 ± 2.0 | 10.6 ± 3.0 |
| melatonin administration | 11 | 5.5 ± 4.5 | 7.2 ± 4.0 |

Table 1 demonstrates that in the non-melatonin administration group, the diapedesis of IgG into cerebral cortex and corpus striatum occurs due to brain ischemia and brain ischemia breaks a blood-brain barrier. It demonstrates also that the breakage of a blood-brain barrier due to brain ischemia is inhibited by the administration of melatonin in the melatonin administration group.

Results of experiment (3) together with those of experiment (2) demonstrate that brain ischemia preferentially injures astroglia leading to the breakage of a blood-brain barrier and that the injury of astroglia, i.e. the breakage of a blood-brain barrier, is inhibited by melatonin.

### (4) Melatonin protects astroglia from the injury caused by a drug (in vitro).

Isolation and incubation of astrocytes were conducted according to the method as described in Neuroscience, 87: 497-807 (1998). The cerebral cortex of a fetal rat was removed, from which astrocytes were separated by trypsin treatment at 37°C for 20 minutes and incubated on a 24 well culture dish containing DMEM media + 10 % FBS under 5% CO₂/air atmosphere at 37°C.

The injury of incubated astrocytes was induced by replacing the medium with a serum-free DMEM medium (serum-free group) or adding 6 mM of a metabolic inhibitor, 3-nitropropionic acid (3-NPA), to the medium (3-NPA group). The serum-free group and the 3-NPA group were divided into two sub-groups, respectively. To one sub-group of each group, 10 µg/ml of melatonin was added to the medium.

The number of the alive astrocytes was counted according to the neutron red assay after 3 days from the incubation in the 3-NPA group or according to the MTT (3-(4,5-dimethyl-thiozolyl)-2,5-diphenyl-tetrazolium bromide) assay after 10 days from the replacement of the medium. The test of significance was conducted by ANOVA. Results are shown in Table 2.

**Table 2**

| group | number of strocytes (% of control; average ± SE) |
|---|---|
| non-treatment | 100 ± 5 |
| 3-NPA | 50 ± 5 |
| 3-NPA + melatonin | 63 ± 4 * |
| serum-free | 34 ± 3 |
| serum-free + elatonin | 48 ± 4 ** |

| | |
|---|---|
| * : p<0.05 (v.s. control) | |
| ** : p<0.01 (v.s. control) | |

As clear from the results in Table 2, melatonin protected astrocytes from the injury caused by 3-NPA or the injury caused by the replacement of a serum-free medium.

In conclusion, the experiments of Example 1 demonstrate that brain ischemia preferentially injures astroglia, destroys blood-brain barrier, and deteriorates vascular permeability, which deterioration causes brain edema. The experiments further demonstrate that the ischemic or poisonous injury of astroglia can be prevented and cured by the administration of melatonin, strongly suggesting that melatonin will treat and prevent brain edema caused by the first injury of astroglia or the second injury of astroglia caused by edema.

### Example 2

### Treatment/prevention of brain edema by melatonin administration (1)

A plug is inserted from an internal carotid artery of a 8 to 10-week-old SD male rat so as to arrive to a branch of its middle cerebral artery, thereby the middle cerebral artery is occluded for 60 minutes.

Astroglias in a penumbra area of brain capillary is injured by brain ischemia and astroglias in other areas and neutron are injured by an ischemic metabolic disorder so that cytotoxic edema is mainly caused. Thereafter, the blood flow is recanalized by removing the plug, thereby a blood flows into the ischemia area at a stroke and moisture is passed from an injured blood-brain barrier to a paremchyma of brain so that vasogenic edema is mainly caused.

Immediately, 6 hours and 12 hours after the recanalization, 0.1 to 1 mg/kg of melatonin is intravenously administered. Extent of brain edema is observed after 24 hours from the recanalization.

The extent of brain edema can be observed by determining the change in intracerebral moisture content using magnetic resonance imaging (MRI) according to two analysis methods; the T₂ highlighted image mainly catches vasogenic edema and the scattering highlighted image mainly catches cytotoxic edema. It can be determined also by observing a brain specimen under a microscope.

In addition, mortality and a cerebral infarction area after orally administrating melatonin in an amount of 6 mg/kg/day for one week are determined.

### Example 3

### Treatment/prevention of brain edema by melatonin administration (2)

### Methods

Ischemia: Twenty male Wistar rats, weighing 250-300g, were randomly assigned to melatonin treatment group and saline treatment group (N=10 per group). Rats of the two groups were subjected to middle cerebral artery (MCA) occlusion/reperfusion surgery. The ischemic surgery was the same as described in Borlongan CV, et al., FASEB J. 14, 1307-1317 (2000) and Nishino H. et al., Neurobiology 2, 223-234 (1994) (see also Examples 1 and 2). Briefly, the rats were anesthetized with halothane and inserted with an embolus (a 4-0 Ethicon nylon filament, with tip diameter tapered to the size of a 26G needle) from the external carotid artery, via the internal carotid artery, to the base of either left or right MCA, thereby the blood flow to the MCA was stopped. During the ischemia, anesthesia was cut off and rats were allowed to recover for 1 hour. Behavioral tests (see below) were performed to confirm successful unilateral ischemia. After 1 hour of the ischemia, the rats were re-anesthetized with halothane, the embolus withdrawn, the blood reperfusion established, and the skin sutured.

Behavioral Tests: The forelimb akinesia test and the spontaneous rotational test were conducted during MCA occlusion (i.e., during the 1 hour-embolism) as the behavioral tests. Detailed description and specific criteria for each test to determine successful ischemia have been reported in Borlongan CV, et al. and Nishino H, et al. supra. Briefly, the forelimb akinesia test involved holding the animal by the tail and noting the positions of the forelimbs. Rats with MCA occlusion demonstrate a characteristic posture; i.e. the forelimb in the ischemic side is stretched out while the forelimb in the contralateral side clipped to their chest muscles. For the spontaneous rotational test, the rat was placed in a box made of Plexiglas and the direction of the rat's rotation was noted over 5 minutes.

Treatment: Melatonin was dissolved in saline and administered orally (6.0 mg/kg) just prior to the 1 hour-MCA occlusion and 1 day after the surgery (melatonin treatment group). Saline was administered similarly to the melatonin non-treatment group.

MRI: A MRI system (SMIS, UK), consisted of a 4.7 T/400 horizontal superconducting magnet equipped with actively shielded gradient coils (60 mT/m, 26-cm i.d.), was used to obtain brain images of ischemic rats. For the determination of the volume of edema, 30 consecutive coronal brain images were obtained using a T₂-weighted multislice spin-echo pulse sequence (TR = 15,000 ms, TE = 80 ms, FOV = 19.2 mm x 19.2 mm, matrix = 64 x 64, slice thickness = 0.6 mm, number of slices = 30, slice gap between slice-to-slice = 0 mm, slice interleave = 5, number of experiments = 2). Volume of edema is (number of pixels in edema region) x (voxel volume), where the voxel volume is 0.054 mm³. Criteria of pixels within the edema region was defined as pixels that showed more than 120% of the signal intensity compared to the averaged signal intensity in contralateral side of the brain. Pixels that corresponded to the ventricles were excluded carefully. Statistics were made by ANOVA followed by Scheffe's t-test. Differences were considered significant at P < 0.05.

### Results

Increases in T₂-weighted MR signals representing edema in MCA occlusion rats were found mainly in the striatum and in the cerebral cortex (Figs. 3 and 4). The volume of cerebral edema was greatly and significantly reduced by the oral administration of melatonin (reduced by 40.8%, P < 0.01, 446.5 33.6 mm³ in the melatonin non-treatment group and 264.2 52.2 mm3 in the melatonin treatment group) (Figs 3, 4 and 5). The protective effect of melatonin against edema was more clearly observed in the cerebral cortex (reduced by 50.3%, P < 0.01, 303.6 22.5 mm³ in the melatonin non-treatment group and 151.0 37.4 mm³ in the melatonin treatment group) (Figs. 3, 4 and 5).

In conclusion, it is clearly demonstrated by the MRI that administration of melatonin is effective to reduce edema, especially in the cerebral cortex in ischemic animals.

## Claims

1. Use of melatonin in the manufacture of a composition for the treatment or prevention of brain edema.

2. A use according to claim 1, wherein the composition is a pharmaceutical composition.

3. A use according to claim 2, wherein melatonin is encapsulated in an encapsulating matrix or a liposome.

4. A use according to claim 2, wherein the composition is for oral administration.

5. A use according to claim 1, wherein the composition is a food composition.

6. A use according to claim 5, wherein melatonin is added to a food in the form of a mixture of melatonin and a food additive.

7. A use according to any one of claims 1 to 6, wherein the composition is for use in the treatment of a subject who is suffering from brain edema.

8. A use according to any one of claims 1 to 6, wherein the composition is for administration to a subject who is at risk of suffering from brain ischemia.

9. A use according to claim 8 wherein the subject at risk of suffering from brain ischemia is a subject suffering from cerebral thrombosis, cerebral embolism, cerebral infarction, cerebral hemorrhage, subarachnoid hemorrhage, transient brain ischemia, hyperlipemia, hypertension, cardiac arrest or brain contusion.

## Patentansprüche

1. Verwendung von Melatonin bei der Herstellung einer Zusammensetzung für die Behandlung oder Vorbeugung von Hirnödemen.

2. Verwendung nach Anspruch 1, worin die Zusammensetzung eine pharmazeutische Zusammensetzung ist.

3. Verwendung nach Anspruch 2, worin Melatonin in eine Einkapselungsmatrix oder ein Liposom eingekapselt ist.

4. Verwendung nach Anspruch 2, worin die Zusammensetzung für die orale Verabreichung bestimmt ist.

5. Verwendung nach Anspruch 1, worin die Zusammensetzung eine Nahrungsmittelzusammensetzung ist.

6. Verwendung nach Anspruch 5, worin Melatonin einem Nahrungsmittel in der Form eines Gemischs aus Melatonin und einem Lebensmittelzusatz zugesetzt ist.

7. Verwendung nach einem der Ansprüche 1 bis 6, worin die Zusammensetzung zur Verwendung bei der Behandlung eines an einem Hirnödem leidenden Patienten bestimmt ist.

8. Verwendung nach einem der Ansprüche 1 bis 6, worin die Zusammensetzung zur Verabreichung an einen Patienten mit Risiko für Hirnischämie bestimmt ist.

9. Verwendung nach Anspruch 8, worin der Patient mit Risiko für Hirnischämie ein Patient ist, der an Hirnthrombose, Hirnembolie, Hirninfarkt, Hirnblutung, Subarachnoidalblutung, vorübergehender Hirnischämie, Hyperlipämie, Hypertonie, Herzstillstand oder Hirnkontusion leidet.

## Revendications

1. Utilisation de la mélatonine dans la fabrication d'une composition pour le traitement ou la prévention de l'oedème cérébral.

2. Une utilisation selon la revendication 1, dans laquelle la composition est une composition pharmaceutique.

3. Une utilisation selon la revendication 2, dans laquelle la mélatonine est encapsulée dans une matrice d'encapsulement ou un liposome.

4. Une utilisation selon la revendication 2, dans laquelle la composition est pour une administration orale.

5. Une utilisation selon la revendication 1, dans laquelle la composition est une composition alimentaire.

6. Une utilisation selon la revendication 5, dans laquelle la mélatonine est ajoutée à un aliment sous la forme d'un mélange de mélatonine et d'un additif alimentaire.

7. Une utilisation selon l'une quelconque des revendications 1 à 6, dans laquelle la composition est pour une utilisation dans le traitement d'un sujet qui souffre d'un oedème cérébral.

8. Une utilisation selon l'une quelconque des revendications 1 à 6, dans laquelle la composition est pour une administration à un sujet qui présente un risque d'éprouver une ischémie cérébrale.

9. Une utilisation selon la revendication 8 dans laquelle le sujet présentant un risque d'éprouver une ischémie cérébrale est un sujet éprouvant une thrombose cérébrale, une embolie cérébrale, un infarctus cérébral, une hémorragie cérébrale, une hémorragie subarachnoïde, une ischémie cérébrale transitoire, une hyperlipémie, une hypertension, un arrêt cardiaque ou une contusion cérébrale.
